# EUROPEAN PATENT APPLICATION

(11) **EP 4 835 859 A2**
(43) Date of publication of application: **04.11.2026**
(21) Application number: 26197594.0
(22) Date of filing: 18.03.2025
(51) Int. Cl.: G08C 17/02

(54) **SENSOR APPARATUS, TERMINAL APPARATUS, AND SENSOR SYSTEM**

(30) Priority: 25.03.2024 JP 2024048460
(62) Document number(s) of the earlier application(s) in accordance with Art. 76 EPC: 25164321.9 / 4 636 387
(71) Applicant: Yokogawa Electric Corporation, Musashino-shi, Tokyo 180-8750 (JP)
(72) Inventor: OKADA, Syuhei, Musashino-shi, Tokyo, 180-8750 (JP)
(74) Representative: Stöckeler, Ferdinand

(57) **Abstract**

The detection of corrosion inside a measurement target and the effective notification of detection results are enabled in a simple configuration. A sensor apparatus (10) includes an activation transmitter (13) configured to transmit an activation signal that is a signal indicating activation of the sensor apparatus (10), and a timer (16) configured to determine transmission timings of transmitting the activation signal from the activation transmitter (13). The activation transmitter (13) is configured to transmit the activation signal at the transmission timings determined by the timer (16). The timer (16) is configured to reduce an interval between the transmission timings in response to variation in the resistance value of a resistor (167) whose resistance value varies in response to the progress of corrosion.

## Description

### CROSS-REFERENCE TO RELATED APPLICATION

This application claims priority to Japanese Patent Application No. 2024-048460, filed on March 25, 2024, the entire contents of which are incorporated herein by reference.

### TECHNICAL FIELD

The present disclosure relates to a sensor apparatus, a terminal apparatus, and a sensor system.

### BACKGROUND

Technology for measuring the internal state of a measurement target in a nondestructive manner is known. For example, Patent Literature (PTL) 1 describes, in a soil retaining structure constructed using a reinforcement material, a safety evaluation method for the soil retaining structure using a corrosion sensor, which detects corrosion of the reinforcement material, and radio frequency identification (RFID).

### CITATION LIST

### Patent Literature

PTL 1: JP 2020-084542 A

### SUMMARY

However, conventional configurations have room for improvement in terms of effectively notifying detection results when corrosion has been detected inside measurement targets.

It would be helpful to allow corrosion inside a measurement target to be detected and detection results to be effectively notified, in a simple configuration.
(1) A sensor apparatus according to some embodiments includes:
   an activation transmitter configured to transmit an activation signal that is a signal indicating activation of the sensor apparatus; and
   a timer configured to determine transmission timings of transmitting the activation signal from the activation transmitter,
   wherein
   the activation transmitter is configured to transmit the activation signal at the transmission timings determined by the timer, and
   the timer is configured to reduce an interval between the transmission timings in response to variation in a resistance value of a resistor whose resistance value varies in response to the progress of corrosion.

As described above, the sensor apparatus reduces the interval between the transmission timings of transmitting the activation signal from the activation transmitter, in response to the variation in the resistance value of the resistor whose resistance value varies in response to the progress of corrosion. Therefore, it is possible to notify an apparatus that receives the activation signal from the sensor apparatus of the degree of corrosion around the sensor apparatus, using a reception interval of the activation signal.

In one embodiment,
(2) the sensor apparatus according to (1) may further include a sensor configured to acquire a measurement value of a surrounding physical quantity,
wherein the activation transmitter may be configured to transmit, as the activation signal, a signal containing the measurement value acquired by the sensor.

As described above, the sensor apparatus transmits, as the activation signal, the signal containing the measurement value acquired by the sensor, and therefore can notify the apparatus that receives the activation signal of the measurement value of the surrounding physical quantity.

In one embodiment,
(3) in the sensor apparatus according to (2), the activation transmitter may be configured to transmit, as the activation signal, a signal containing the measurement value acquired by the sensor immediately before transmission of the signal.

As described above, the activation transmitter transmits, as the activation signal, the signal containing the measurement value acquired by the sensor immediately before transmission of the signal. Therefore, the sensor only needs to perform measurement immediately before the activation transmitter transmits the activation signal, which allows reduction in power consumption of the sensor apparatus.

In one embodiment,
(4) in the sensor apparatus according to any one of (1) to (3), the activation transmitter may be configured to transmit the activation signal by wireless communication.

As described above, the activation transmitter transmits the activation signal by wireless communication, which allows reduction in installation cost of the sensor apparatus.

In one embodiment,
(5) the sensor apparatus according to any one of (1) to (4) may further include:
a corrosion transmitter configured to transmit, at the transmission timings by wireless communication, a corrosion signal that is a signal indicating that the corrosion has progressed to a certain degree; and
a shield configured to block the corrosion signal transmitted from the corrosion transmitter,
wherein the shield may be configured to have a reduced function of blocking the corrosion signal in response to the progress of the corrosion.

As described above, the sensor apparatus blocks the corrosion signal using the shield, which has the reduced function of blocking the corrosion signal in response to the progress of the corrosion. Therefore, the sensor apparatus can notify an apparatus communicable with the sensor apparatus that the corrosion around the sensor apparatus is progressing, by reception of the corrosion signal.

In one embodiment,
(6) in the sensor apparatus according to (5), the shield may be made of iron.

As described above, the shield is made of iron, so the sensor apparatus can be configured inexpensively.

In one embodiment,
(7) the sensor apparatus according to any one of (1) to (6) may further include:
a rechargeable battery configured to supply electric power to drive the timer; and
a power receiver configured to receive electric power to recharge the battery.

As described above, the sensor apparatus is driven by the rechargeable battery, and therefore can be installed in a variety of environments, regardless of location.

(8) A sensor apparatus according to some embodiments includes:
a corrosion transmitter configured to transmit, at predetermined transmission timings by wireless communication, a corrosion signal that is a signal indicating that corrosion has progressed to a certain degree; and
a shield configured to block the corrosion signal transmitted from the corrosion transmitter,
wherein the shield is configured to have a reduced function of blocking the corrosion signal in response to the progress of the corrosion.

As described above, the sensor apparatus blocks the corrosion signal using the shield, which has the reduced function of blocking the corrosion signal in response to the progress of the corrosion. Therefore, the sensor apparatus can notify the apparatus communicable with the sensor apparatus that the corrosion around the sensor apparatus is progressing, by reception of the corrosion signal.

In one embodiment,
(9) in the sensor apparatus according to (8), the shield may be made of iron.

As described above, the shield is made of iron, so the sensor apparatus can be configured inexpensively.

In one embodiment,
(10) the sensor apparatus according to (8) or (9) may further include:
a rechargeable battery configured to supply electric power to drive the corrosion transmitter; and
a power receiver configured to receive electric power to recharge the battery.

As described above, the sensor apparatus is driven by the rechargeable battery, and therefore can be installed in a variety of environments, regardless of location.

A terminal apparatus according to some embodiments is
(11) a terminal apparatus configured to be communicable with the sensor apparatus according to any one of (1) to (7), the terminal apparatus including a controller configured to cause an output interface to output an alert when a reception interval of the activation signal from the sensor apparatus is less than a predetermined threshold value.

As described above, the terminal apparatus causes the output interface to output the alert when the reception interval of the activation signal is less than the predetermined threshold value, and therefore can effectively notify a user that the corrosion around the sensor has progressed.

A terminal apparatus according to some embodiments is
(12) a terminal apparatus configured to be communicable with the sensor apparatus according to any one of (8) to (10), the terminal apparatus including a controller configured to cause an output interface to output an alert upon receiving the corrosion signal from the sensor apparatus.

As described above, the terminal apparatus causes the output interface to output the alert upon receiving the corrosion signal from the corrosion transmitter, which is shielded by the shield, and therefore can effectively notify a user that the corrosion around the sensor has significantly progressed.

A terminal apparatus according to some embodiments is
(13) a terminal apparatus configured to be communicable with the sensor apparatus according to (7) or (10), the terminal apparatus including a controller configured to cause a power supply to supply electric power to the sensor apparatus when no signal has been received from the sensor apparatus over a predetermined period of time.

As described above, the terminal apparatus starts supplying the electric power when no signal has been received from the sensor apparatus, which is driven by the rechargeable battery, over the predetermined period of time, and therefore can recharge the sensor apparatus at an appropriate timing.

A sensor system according to some embodiments is
(14) a sensor system including:
the sensor apparatus according to any one of (1) to (7); and
the terminal apparatus according to (11).

As described above, in the sensor system, the sensor apparatus reduces the interval between the transmission timings of transmitting the activation signal from the activation transmitter, in response to the variation in the resistance value of the resistor whose resistance value varies in response to the progression of corrosion. Therefore, it is possible to notify an apparatus that receives the activation signal from the sensor apparatus of the degree of corrosion around the sensor apparatus, using a reception interval of the activation signal.

A sensor system according to some embodiments is
(15) a sensor system including:
the sensor apparatus according to any one of (8) to (10); and
the terminal apparatus according to (12).

As described above, in the sensor system, the sensor apparatus blocks the corrosion signal using the shield, which has the reduced function of blocking the corrosion signal in response to the progress of the corrosion. Therefore, the sensor apparatus can notify an apparatus communicable with the sensor apparatus that the corrosion around the sensor apparatus is progressing, by reception of the corrosion signal.

According to one embodiment of the present disclosure, it is possible to detect corrosion inside a measurement target and effectively notify detection results, in a simple configuration.

### BRIEF DESCRIPTION OF THE DRAWINGS

In the accompanying drawings:
FIG. 1 is a diagram illustrating an example configuration of a sensor system according to an embodiment;
FIG. 2 is a block diagram illustrating an example configuration of a sensor apparatus in FIG. 1;
FIG. 3 is a circuit diagram illustrating an example configuration of a timer in FIG. 2;
FIG. 4 is a graph illustrating example operations of a first transmitter in FIG. 2;
FIG. 5 is a graph illustrating example operations of a second transmitter in FIG. 2;
FIG. 6 is a block diagram illustrating an example configuration of a terminal apparatus in FIG. 1; and
FIG. 7 is a flowchart illustrating example operations of the terminal apparatus in FIG. 1.

### DETAILED DESCRIPTION

### <Comparative Example>

The configuration of a comparative example (claim 1 of PTL 1) is

"A safety evaluation method for a soil retaining structure constructed using a reinforcement material, the safety evaluation method comprising at least:
installing, in the reinforcement material, at least one corrosion sensor that detects corrosion;
installing, in soil or a wall panel, a radio frequency identification (RFID) tag that collects information from the corrosion sensor and outputs data in an RFID method; and
acquiring a measurement value from the RFID tag in the RFID method."

In the configuration according to the comparative example, an operator cannot know the status of corrosion of the reinforcement material unless the operator himself/herself carries out measurement, because information regarding the corrosion of the reinforcement material is acquired in response to the operator's instruction to an apparatus. In addition, the configuration according to the comparative example requires installation of the apparatus that conforms to the RFID method, because the measurement value is acquired in the RFID method. Accordingly, the configuration according to the comparative example has room for improvement in terms of effectively notifying detection results when corrosion inside a measurement target has been detected.

### <Embodiment>

An embodiment of the present disclosure will be described below with reference to the drawings. In the drawings, parts that have the same configurations or functions are indicated with the same reference numerals. In the description of the present embodiment, duplicated descriptions of the same parts may be omitted or simplified as appropriate.

### (Sensor System 1)

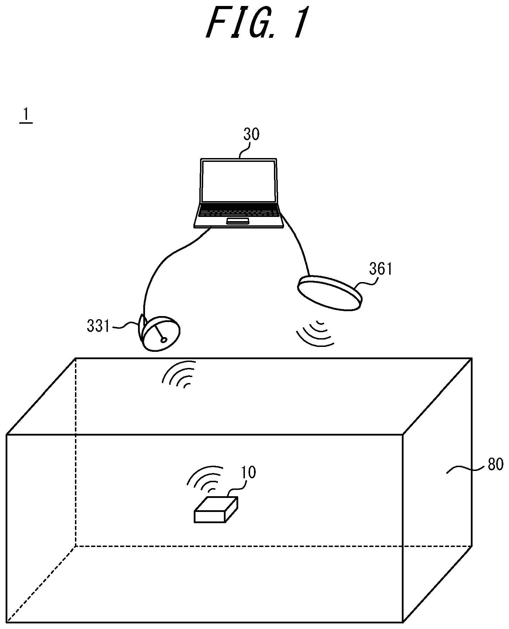

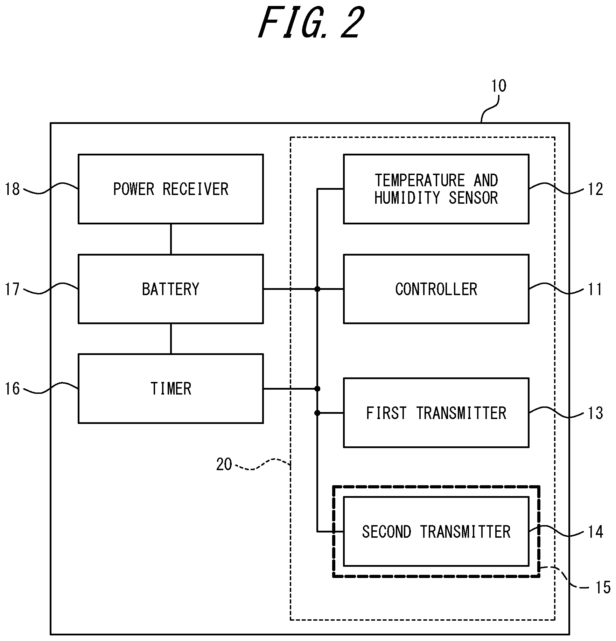

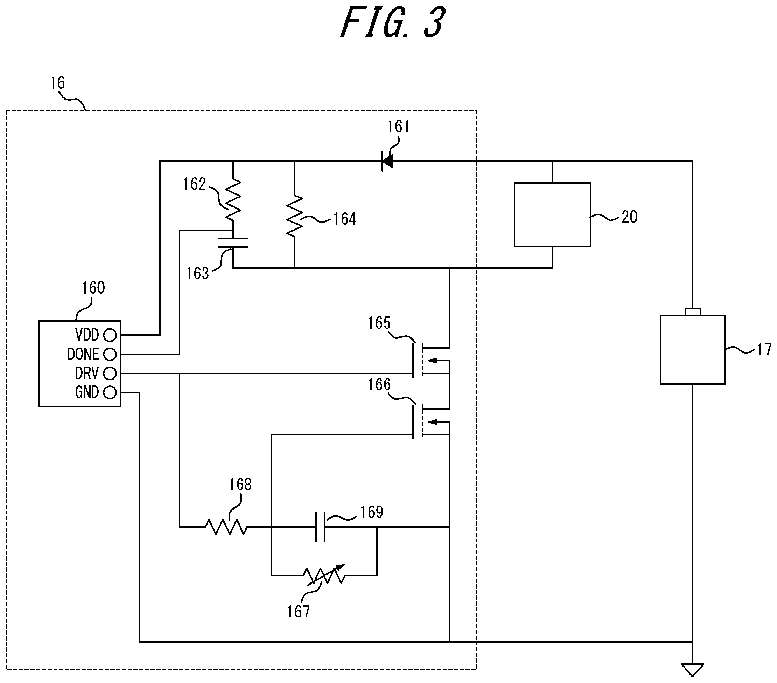

FIG. 1 is a diagram illustrating an example configuration of a sensor system 1 according to the embodiment. The sensor system 1 includes a sensor apparatus 10 and a terminal apparatus 30. The sensor system 1 estimates, for example, corrosion and the like of steel bars and wood around a sensor by detecting corrosion of corrodible parts, such as iron, embedded in the sensor apparatus 10 by measurement of the current temperature and humidity inside a measurement target 80, by a past water intrusion history, and the like. The corrodible parts may include, for example, an environment-dependent resistor 167, a shield 15, and the like as described below (see FIGS. 2 and 3).

The sensor apparatus 10 is installed inside the measurement target 80, which is difficult for an operator to access directly, and detects information regarding water intrusion, corrosive environment, and the like. The measurement target 80 is, for example, any object, including solids, liquids, and gases, that is difficult for humans to enter, in buildings including factories and plants, and any other structures. The sensor apparatus 10 transmits detection results of the information to the terminal apparatus 30. The sensor apparatus 10 transmits signals, including the detection results, to the terminal apparatus 30 by any wireless communication, e.g., Bluetooth^{®} (Bluetooth is a registered trademark in Japan, other countries, or both), a wireless local area network (LAN), or the like. By transmitting the signals using electromagnetic waves in a frequency range with high permeability, the sensor apparatus 10 can allow the signals to reach the terminal apparatus 30 with high reliability, regardless of the presence or absence of obstacles. As a matter of course, the sensor apparatus 10 may be communicably connected to the terminal apparatus 30 with a wired cable. As described below, the sensor apparatus 10 operates using electric power of a rechargeable battery 17 (see FIG. 2).

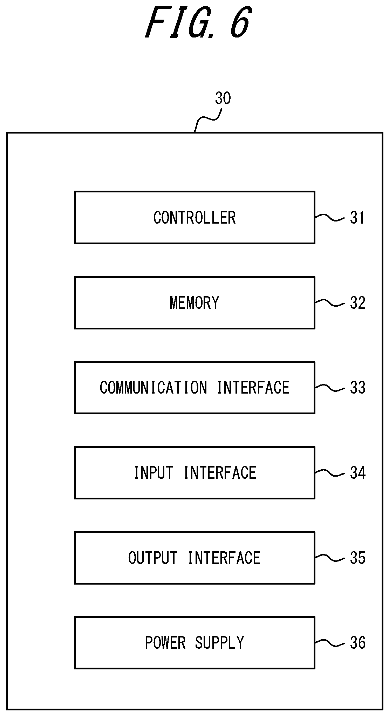

The terminal apparatus 30 notifies the operator of the detection results by the sensor apparatus 10, an alarm, and the like, based on the signals received from the sensor apparatus 10. The terminal apparatus 30 includes a receiver 331 and a wireless power supply 361. The receiver 331 is an example of a communication interface 33 (see FIG. 6) described below, and receives the signals including the detection results transmitted from the sensor apparatus 10. The wireless power supply 361 is an example of a power supply 36 (see FIG. 6) described below, and wirelessly transmits electric power to the sensor apparatus 10 for recharging the battery 17. The terminal apparatus 30 is, for example, a personal computer (PC), but may be a tablet terminal, a smartphone, or any other general-purpose device, or any non-general-purpose device.

### (Sensor Apparatus 10)

FIG. 2 is a block diagram illustrating an example configuration of the sensor apparatus 10 in FIG. 1. As illustrated in FIG. 2, the sensor apparatus 10 includes a measurement unit 20, a timer 16, the battery 17, and a power receiver 18.

The measurement unit 20 measures the information regarding water intrusion, corrosive environment, and the like around the sensor apparatus 10. The measurement unit 20 includes a controller 11, a temperature and humidity sensor 12, a first transmitter 13, a second transmitter 14, and the shield 15.

The controller 11 controls operations of the entire sensor apparatus 10. The controller 11 may include one or more processors and memories. In the present embodiment, the "processor" is a general-purpose processor or a dedicated processor specialized for specific processing, but is not limited to these. The memory includes, for example, any memory module, such as a hard disk drive (HDD), a solid state drive (SSD), a read-only memory (ROM), and a random access memory (RAM). The memory may function as main memory, auxiliary memory, or cache memory. The memory stores any information used in the operations of the sensor apparatus 10.

The temperature and humidity sensor 12 measures ambient temperature and humidity. The temperature and humidity sensor 12 measures the temperature using a temperature sensor integrated circuit (IC), for example, but may measure the temperature using another method such as a resistance thermometer bulb, a thermocouple, or a bimetal. The temperature and humidity sensor 12 measures the humidity using a micro electromechanical systems (MEMS) type capacitive sensor, for example, but may measure the humidity using another method such as an electric resistance type. Note that, the temperature and humidity sensor 12 may be a sensor that measures another physical quantity, e.g., sound, vibration, gas, illuminance, or the like, instead of or in addition to the ambient temperature and humidity.

The first transmitter 13, as an activation transmitter, transmits a signal (first signal) containing measurement values of the physical quantities, such as the temperature and humidity measured by the temperature and humidity sensor 12, to the terminal apparatus 30 in a period during which the timer 16 drives the measurement unit 20. The first signal functions as an activation signal indicating activation of the sensor apparatus 10. The first transmitter 13 transmits the signal containing the measurement values of the temperature and humidity by wireless communication, but may transmit the signal by wired communication.

The second transmitter 14, as a corrosion transmitter, transmits a predetermined signal (second signal, e.g., dummy data) by wireless communication in the period during which the timer 16 drives the measurement unit 20. The second signal functions as a corrosion signal indicating that corrosion has progressed to a certain degree. The first transmitter 13 and the second transmitter 14 may transmit signals that are different from each other, and the terminal apparatus 30 that has received the signals may be able to identify which of the first transmitter 13 and the second transmitter 14 has transmitted which of the signals. For example, the first transmitter 13 and the second transmitter 14 may transmit the signals that contain header information indicating which of the first transmitter 13 and the second transmitter 14 is the sender of the signal.

The shield 15 shields the surroundings of the second transmitter 14, and blocks the wireless signal transmitted from the second transmitter 14. The shield 15 has a reduced function of blocking the wireless signal from the second transmitter 14, in response to the progress of corrosion. For example, the shield 15 may be composed of iron or other materials that corrode due to ambient environmental conditions, including temperature and humidity. Therefore, when the environment around the sensor apparatus 10 is corrosive, the shield 15 corrodes with a lapse of time. As the degree of corrosion of the shield 15 progresses, holes appear in the shield 15, and the terminal apparatus 30 receives the signal transmitted from the second transmitter 14. Alternatively, when an anomaly such as a change or crack occurring in an insulator around the second transmitter 14 occurs, the terminal apparatus 30 receives the signal from the second transmitter 14. Therefore, the terminal apparatus 30 can detect that corrosion is progressing around the sensor apparatus 10, by receiving the signal transmitted from the second transmitter 14.

The timer 16 drives the measurement unit 20, which includes the first transmitter 13 and the second transmitter 14, in cycles corresponding to the degree of corrosion of the environment-dependent resistor 167 (see FIG. 3) described below. As a result, the first transmitter 13 transmits the signal at a frequency corresponding to the degree of corrosion of the environment-dependent resistor 167. Therefore, the terminal apparatus 30 can not only acquire the measurement values of the temperature and humidity from the signal transmitted from the first transmitter 13, but also detect the degree of corrosion around the sensor apparatus 10 based on the frequency of reception of the signal.

The battery 17 supplies electric power to drive each component of the sensor apparatus 10. The battery 17 may be a rechargeable battery, such as a lithium-ion battery, a sodium-ion battery, or a potassium-ion battery.

The power receiver 18 receives electric power transmitted from the power supply 36 (e.g., wireless power supply 361) of the terminal apparatus 30, and recharges the battery 17.

FIG. 3 is a circuit diagram illustrating an example configuration of the timer 16 in FIG. 2. As illustrated in FIG. 3, the timer 16 includes a timer IC 160, a diode 161, resistors 162, 164, and 168, capacitors 163 and 169, switches 165 and 166, and the environment-dependent resistor 167. As illustrated in FIG. 3, the timer 16 is connected to the measurement unit 20 and the battery 17.

The timer IC 160 has a VDD terminal, a DONE terminal, a DRV terminal, and a GND terminal. The VDD terminal is an input terminal to which a positive terminal of the battery 17 is connected. The DONE terminal is an input terminal to which a signal of LOW or HIGH is input. The DRV terminal is an output terminal from which a direct current (DC) signal of LOW or HIGH is output. The GND terminal is an input terminal to which a ground (e.g., a negative terminal of the battery 17) is connected. Upon switching the input signal to the DONE terminal from LOW to HIGH, the timer IC 160 sets the output signal of the DRV terminal to LOW. The timer IC 160 switches the output signal of the DRV terminal from LOW to HIGH after a lapse of a predetermined fixed period of time (first time) since the HIGH signal has been input to the DONE terminal. The timer IC 160 may be configured with any commercially available timer with such a function.

The timer IC 160 is provided with a counter that counts a count value by a clock signal of a predetermined frequency, and the first time may be measured by the fact that the count value of the counter has reached a predetermined value. In this case, the timer IC 160 may determine that the input signal to the DONE terminal has switched from LOW to HIGH when the voltage of the input signal to the DONE terminal exceeds a predetermined threshold value. Upon determining that the input signal to the DONE terminal has switched to HIGH, the timer IC 160 may forcibly set the output signal of the DRV terminal to LOW and reset the count value of the counter.

In the configuration illustrated in FIG. 3, when both the switches 165 and 166 are turned on, the measurement unit 20 is driven by voltage applied from the battery 17. The condition for the switch 165 to be turned on is that the output signal from the DRV terminal of the timer IC 160 is HIGH. The condition for the switch 166 to be turned on is that electric charge accumulates in the capacitor 169 and the voltage between terminals of the capacitor 169 becomes sufficiently high. Therefore, the condition for the measurement unit 20 to be driven is that the output signal from the DRV terminal of the timer IC 160 is HIGH and that the voltage between the terminals of the capacitor 169 is sufficiently high. In the configuration in FIG. 3, the switches 165 and 166 are n-type metal-oxide-semiconductor field-effect transistors (MOSFETs), but may be configured using another method, such as mechanical relays.

In the timer 16, the diode 161, the resistors 162, 164, and 168, the capacitors 163 and 169, and the switches 165, 166 are highly resistant to the environment. The electrical characteristics of these devices hardly vary due to the temperature, humidity, and the like around the sensor apparatus 10. On the other hand, the environmental-dependent resistance 167 is not resistant to the environment and gradually deteriorates. Specifically, the resistance value of the environmental-dependent resistance 167 gradually increases due to the effects of the temperature, humidity, and the like around the sensor apparatus 10.

Operations of the timer IC 16 after output of the DRV terminal of the timer IC 160 is HIGH will be described. When the output signal of the DRV terminal is HIGH, current is continuously supplied from the DRV terminal to the capacitor 169 via the resistor 168. Here, the output voltage of the timer IC 160 is divided according to the resistance values of the resistor 168 and the environment-dependent resistor 167, which are connected in series to each other. Therefore, when the resistance value of the environment-dependent resistor 167 is low due to little corrosion, almost all of the voltage of the DRV terminal is applied to the resistor 168, and only low voltage is applied between the terminals of the capacitor 169. In this case, the gate-source voltage of the switch 166 is insufficient, and the switch 166 is not turned on. In other words, no current flows through the measurement unit 20, and hence the measurement unit 20 does not operate. Note that, even when the resistance value of the environment-dependent resistor 167 is low, electric charge accumulates in the capacitor 169 with a long lapse of time. As a result, when the output of the DRV terminal is HIGH, the switch 166 is turned on with a long lapse of time, and the measurement unit 20 is driven.

When the resistance value of the environment-dependent resistor 167 increases due to corrosion, the voltage between the terminals of the capacitor 169 increases. When the DRV terminal of the timer IC 160 continues outputting the HIGH signal for a fixed period of time, the voltage between the terminals of the capacitor 169 exceeds a voltage threshold value required to turn the switch 166 on, and the switch 166 is turned on. In this case, the switches 165 and 166 are both turned on at the same time, so the measurement unit 20 is driven. The time required for the voltage between the terminals of the capacitor 169 to exceed the voltage threshold value depends on the resistance value of the environment-dependent resistor 167. The higher the resistance value of the environment-dependent resistor 167, i.e., the more the corrosion progresses, the faster the voltage between the terminals of the capacitor 169 reaches the voltage threshold value to turn the switch 166 on. The time (second time) from when the output of the DRV terminal of the timer IC 160 switches from LOW to HIGH to when the switch 166 is turned on becomes shorter as the resistance value of the environment-dependent resistor 167 increases.

When both the switches 165 and 166 are turned on, current flows through the diode 161, which is connected to the positive terminal of the battery 17, to the resistors 164 and 162 and the capacitor 163 too. In response to this, electric charge accumulates in the capacitor 163, and the voltage between terminals of the capacitor 163 increases. When the voltage between the terminals of the capacitor 163 exceeds a predetermined threshold value after a fixed period of time, the timer IC 160 determines that the input signal to the DONE terminal of the timer IC 160 has switched from LOW to HIGH. As a result, the timer IC 160 switches the output signal from the DRV terminal to LOW and resets the counter of the timer IC 160. The time (third time) from when both the switches 165 and 166 are turned on until when the input signal to the DONE terminal switches from LOW to HIGH is a fixed period of time determined by the circuit constants of the circuit including the resistors 162 and 164 and the capacitor 163.

When the output signal of the DRV terminal of the timer IC 160 switches from HIGH to LOW, the electric charge stored between the terminals of the capacitor 163 is discharged via the resistor 164. The diode 161 acts to prevent current from flowing into the measurement unit 20 during the discharge process of the capacitor 163. As a result, input to the DONE terminal becomes zero, and the circuitry (including the diode 161, the resistors 162 and 164, and the capacitor 163) that controls drive time of the measurement unit 20 is reset.

On the other hand, when the output signal of the DRV terminal of the timer IC 160 becomes LOW, the electric charge accumulated in the capacitor 169 is discharged via the environment-dependent resistor 167 and the switch 166. Thereby, the circuitry (including the environment-dependent resistor 167, the resistor 168, and the capacitor 169) that controls the timing to turn the switch 166 on is reset.

After a lapse of the first time again, the output signal of the DRV terminal of the timer IC 160 switches from LOW to HIGH, and the above sequence is repeated.

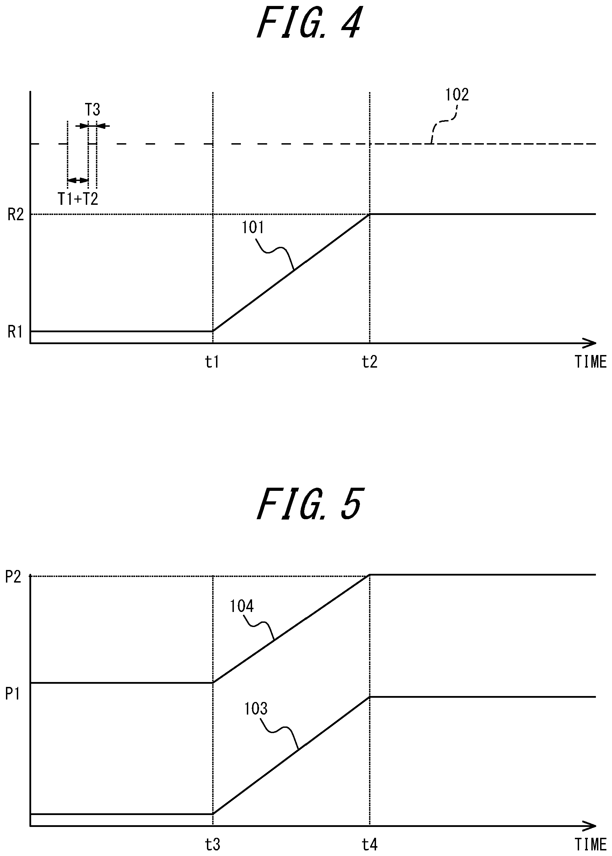

Operations of the first and second transmitters 13 and 14, based on the control of the timer 16 as described above, will be described with reference to FIGS. 4 and 5. FIG. 4 is a graph illustrating example operations of the first transmitter 13 in FIG. 2. FIG. 5 is a graph illustrating example operations of the second transmitter 14 in FIG. 2.

In FIG. 4, the horizontal axis indicates time. The graph 101 illustrates variation in the resistance value of the environment-dependent resistor 167 with time. In the example in FIG. 4, the resistance value of the environment-dependent resistor 167 is R1 and does not vary until a time t1. Between the time t1 and a time t2, the environment-dependent resistor 167 deteriorates, and the resistance value of the environment-dependent resistor 167 increases from R1 to R2 (>R1). At and after the time t2, the resistance value of the environment-dependent resistor 167 is constant at R2.

The graph 102 illustrates the distribution of time during which the measurement unit 20 is continuously driven. The aforementioned third time T3 corresponds to the time (transmission timing) during which the measurement unit 20 is continuously driven. The total time of the first time T1 and the second time T2 (T1 + T2) corresponds to the time during which the measurement unit 20 stops being driven. Therefore, in the graph 102, straight lines of a length T3 are distributed at intervals of T1 + T2. During the time T3, the sensor apparatus 10 performs measurement using the temperature and humidity sensor 12, transmits the signals from the first transmitter 13 and the second transmitter 14, and the like. The first transmitter 13 may transmit, as the first signal, a signal containing measurement values of the physical quantities measured by the temperature and humidity sensor 12 immediately before transmitting the signal. As described above, the lengths of the time T1 and T3 are constant, but the length of the time T2 decreases in response to the progress of deterioration of the environment-dependent resistor 167. Therefore, between the time t1 and the time t2, the intervals in the graph 102 become narrower with a lapse of time. Thus, the intervals (T1 + T2) at which the measurement unit 20 is driven decrease in response to the degree of deterioration of the environment-dependent resistor 167, so the terminal apparatus 30 can detect the degree of corrosion around the sensor apparatus 10, based on the intervals at which the terminal apparatus 30 receives the signal from the first transmitter 13.

In FIG. 5, the horizontal axis indicates time. The graph 103 schematically illustrates the degree of corrosion of the shield 15. In the example in FIG. 5, the degree of corrosion of the shield 15 does not vary until a time t3. Between the time t3 and a time t4, the corrosion of the shield 15 progresses. At and after the time t4, the degree of corrosion of the shield 15 is constant. At the time t4, the shield 15 is in a state in which the corrosion has progressed so that holes have appeared.

The graph 104 illustrates variation in the strength of the signal from the second transmitter 14 received by the terminal apparatus 30. Up to the time t3, the strength of the signal received from the second transmitter 14 is P1. Between the time t3 and the time t4, the shield 15 corrodes, and the signal from the second transmitter 14 becomes easier to receive by the terminal apparatus 30. Therefore, between the time t3 and the time t4, the strength of the signal received from the second transmitter 14 increases from P1 to P2 (>P1). At and after the time t4, the strength of the signal received from the second transmitter 14 is constant at P2. Since P1 is 0 or an extremely low value, the terminal apparatus 30 can detect, based on the fact that the terminal apparatus 30 has received the signal from the second transmitter 14, that corrosion is significantly progressing around the sensor apparatus 10.

As described above, the resistance value of the environment-dependent resistor 167 increases as corrosion progresses due to the surrounding water or other environmental factors, and as a result, the cycles in which the timer 16 is turned on become shorter. Therefore, the more the sensor apparatus 10 deteriorates due to prolonged exposure to the corrosive environment, the more frequently the terminal apparatus 30 receives the temperature and humidity data transmitted from the first transmitter 13. As the deterioration around the sensor apparatus 10 progresses even further, the terminal apparatus 30 becomes able to communicate with the second transmitter 14 too. Therefore, the sensor apparatus 10 can detect corrosion inside the measurement target and effectively notify the detection results in the simple structure.

### (Terminal Apparatus 30)

FIG. 6 is a block diagram illustrating an example configuration of the terminal apparatus 30 in FIG. 1. As illustrated in FIG. 4, the terminal apparatus 30 includes a controller 31, a memory 32, the communication interface 33, an input interface 34, an output interface 35, and the power supply 36.

The controller 31 includes one or more processors. In the present embodiment, the "processor" is a general-purpose processor or a dedicated processor specialized for specific processing, but is not limited to these. The controller 31 is communicably connected to each component of the terminal apparatus 30, and controls operations of the entire terminal apparatus 30.

The memory 32 includes any memory module, such as an HDD, an SSD, a ROM, and a RAM. The memory 32 may function as main memory, auxiliary memory, or cache memory. The memory 32 stores any information to be used in operations of the terminal apparatus 30. For example, the memory 32 may store a system program, an application program, various information received by the communication interface 33, and the like. The memory 32 is not limited to being built in the terminal apparatus 30, and may be an external database or an external memory module.

The communication interface 33 includes any communication module that can be communicably connected to the sensor apparatus 10 using any communication technology including Bluetooth^{®}. The communication interface 33 may further include a communication control module for controlling communication with the sensor apparatus 10, and a memory module for storing communication data such as identification information required for communication with the sensor apparatus 10.

The input interface 34 includes one or more input interfaces that accept the operator's input operations and acquire input information based on the operator's operations. For example, the input interface 34 is physical keys, capacitive keys, a pointing device, a touch screen that is integrally provided with a display of the output interface 35, a microphone that accepts audio input, or the like, but is not limited to these.

The output interface 35 includes one or more output interfaces that output information to the operator and notify the operator. For example, the output interface 35 is a display that outputs information as images, or a speaker that outputs information as audio, but is not limited to these. Such a display may be, for example, a liquid crystal panel display, an organic electro luminescence (EL) display, or the like. Note that, at least one of the input interface 34 or the output interface 35 described above may be configured integrally with the terminal apparatus 30, or may be provided separately.

The power supply 36 supplies electric power to the sensor apparatus 10. The power supply 36 is realized as the wireless power supply 361, for example, but may supply electric power to the sensor apparatus 10 via a wired cable.

The functions of the terminal apparatus 30 can be achieved by executing a computer program (program) according to the present embodiment on a processor included in the controller 31. In other words, the functions of the terminal apparatus 30 can be achieved by software. The computer program causes a computer to execute processing of steps included in the operations of the terminal apparatus 30, thereby causing the computer to achieve the functions corresponding to the processing of the individual steps. In other words, the computer program is a program to cause the computer to function as the terminal apparatus 30 according to the present embodiment.

Some or all of the functions of the terminal apparatus 30 may be achieved by dedicated circuitry included in the controller 31. In other words, some or all of the functions of the terminal apparatus 30 may be achieved by hardware. The terminal apparatus 30 may be implemented by a single computer, or may be implemented by the cooperation of multiple computers.

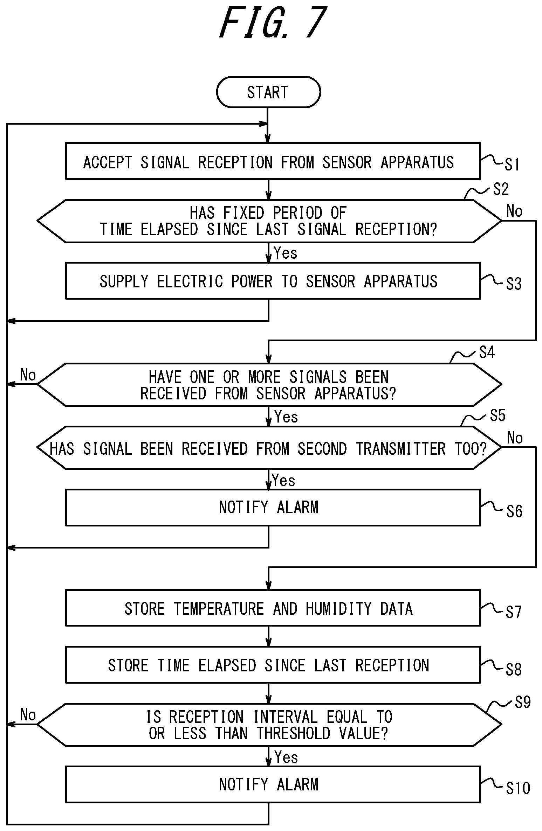

FIG. 7 is a flowchart illustrating example operations of the terminal apparatus 30 in FIG. 1. The operations of the terminal apparatus 30 described with reference to FIG. 7 may correspond to one of control methods of the terminal apparatus 30. The operation of each step in FIG. 7 may be executed based on the control by the controller 31 of the terminal apparatus 30. The following processing is performed in a situation in which the sensor apparatus 10 drives the measurement unit 20 at predetermined timings, as illustrated by the graph 102 in FIG. 4.

In step S1, the controller 31 accepts a signal reception from the sensor apparatus 10 via the communication interface 33.

In step S2, the controller 31 determines whether a predetermined fixed period of time has elapsed since the last signal reception from the sensor apparatus 10. This fixed period of time may be a value greater than the maximum value of the total time (T1 + T2) of the aforementioned first and second times. When the fixed period of time has elapsed (step S2: Yes), the operation proceeds to step S3. When the fixed period of time has not elapsed (step S2: No), the operation proceeds to step S4.

In step S3, the controller 31 supplies electric power to the sensor apparatus 10 via the power supply 36. Therefore, when the terminal apparatus 30 has been unable to confirm the signal reception from the sensor apparatus 10 for the fixed period of time, the terminal apparatus 30 can automatically start recharging the sensor apparatus 10. When the power supply to the sensor apparatus 10 is terminated, the controller 31 returns to step S1.

In step S4, the controller 31 determines whether one or more signals have been received from the sensor apparatus 10. When the controller 31 has received the one or more signals (step S4: Yes), the operation proceeds to step S5. When the controller 31 has not received the one or more signals (step S4: No), the operation returns to step S1.

In step S5, the controller 31 determines whether the one or more signals received in step S4 are only from the first transmitter 13 of the sensor apparatus 10, or include a signal from the second transmitter 14. When the one or more signals include a signal from the second transmitter 14 (step S5: Yes), the operation proceeds to step S6. When the one or more signals do not include a signal from the second transmitter 14 (step S5: No), the operation proceeds to step S7.

In step S6, the controller 31 notifies an alarm via the output interface 35. The terminal apparatus 30 receives the signal from the second transmitter 13 when corrosion of the shield 15 of the sensor apparatus 10 has progressed. The controller 31 then notifies an operator of information indicating that corrosion of the sensor apparatus 10 is significantly progressing, by displaying the information on a display of the output interface 35 or by audibly outputting the information from a speaker of the output interface 35. After completing the processing in step S6, the operation returns to step S1.

In step S7, the controller 31 stores, in the memory 32, temperature and humidity data, including temperature and humidity measurement values, contained in the signal received from the first transmitter 13 in step S4.

In step S8, the controller 31 stores, in the memory 32, the time elapsed since the last time when the signal from the first transmitter 13 has been received until the time when the signal has been received in step S4.

In step S9, the controller 31 determines whether the reception interval stored in step S8 is equal to or less than a predetermined threshold value. When the reception interval stored in step S8 is equal to or less than the threshold value (step S9: Yes), the operation proceeds to step S10. When the reception interval stored in step S8 is not equal to or less than the threshold value (step S9: No), the operation returns to step S1.

In step S10, the controller 31 notifies an alarm via the output interface 35. As described above, the interval between which the signal is transmitted from the first transmitter 13 becomes shorter as corrosion of the environment-dependent resistor 167 of the timer 16 progresses. Therefore, the controller 31 notifies the operator of information indicating that corrosion of the sensor apparatus 10 is progressing, by displaying the information on a display of the output interface 35 or by audibly outputting the information from a speaker of the output interface 35. After completing the processing in step S10, the operation returns to step S1.

As described above, the terminal apparatus 30 detects the presence or absence of deterioration around the sensor apparatus 10 and the degree thereof, based on the reception interval of the signal from the first transmitter 13 of the sensor apparatus 10 and the presence or absence of reception of the signal from the second transmitter 14. Upon detecting deterioration, the terminal apparatus 30 notifies the operator of the alarm. Therefore, the operator can easily recognize the deterioration of the measurement target 80 without having to perform measurement by himself/herself.

Note that, the signal from the second transmitter 14 is received when the degree of corrosion is significant, such as when holes appear in the shield 15. Therefore, in step S6, the controller 31 may notify a more emphatic alarm than the alarm in step S10. For example, in step S6, the controller 31 may display a more conspicuous image, display information prompting the replacement of the sensor apparatus 10, or output audio or vibration together with display of the image.

As described above, for the measurement target 80 inside a solid, a gas, or the like, the sensor system 1 detects information regarding moisture intrusion into a part that is difficult for an operator to access environmentally and corrosive environment including water, temperature, and humidity. Specifically, the sensor apparatus 10 includes the measurement unit 20 having the first transmitter 13 that transmits a signal, and the timer 16 that determines multiple drive periods during which the measurement unit 20 is driven. Here, the first transmitter 13 transmits the signal during the multiple drive periods (transmission timings) determined by the timer 16. The timer 16 reduces the intervals between the multiple drive periods (transmission timings) in response to variation in the resistance value of the environment-dependent resistor 167, which is a resistor whose resistance value varies in response to the progress of corrosion.

Accordingly, the sensor system 1 can detect corrosion inside the measurement target 80 and effectively notify detection results in the simple configuration, without having to install a special reader such as an RFID reader on the operator's side or a specially processed part on the sensor's side. The sensor apparatus 10 is configured to transmit signals by wireless communication from the first transmitter 13 and the second transmitter 14 and be driven by the battery 17, thus being able to perform nondestructive inspections on various measurement targets 80. Note that, in the present embodiment, the resistance value of the environment-dependent resistor 167 increases in response to the progress of corrosion, but the sensor apparatus 10 may be provided with, as the environment-dependent resistor 167, a resistor whose resistance value decreases in response to the progress of corrosion.

In the sensor system 1, the terminal apparatus 30 can detect corrosive environment around the sensor apparatus 10 due to water or other environmental factors, based on the frequency of reception of a signal from the first transmitter 13 and the presence or absence of a signal from the second transmitter 14. The sensor apparatus 10 transmits, from the first transmitter 13, the signal that includes at least one of a temperature measurement value or a humidity measurement value measured by the temperature and humidity sensor 12. Therefore, the sensor system 1 can effectively notify not only the measurement values of the physical quantities including temperature and humidity, but also information regarding the corrosive environment around the sensor apparatus 10 due to water or other environmental factors.

The sensor apparatus 10 has a simple configuration, and therefore can be manufactured compactly and inexpensively. The measurement unit 20 of the sensor apparatus 10 operates only in multiple drive periods, during which the measurement unit 20 is driven, determined by the timer 16, so the sensor apparatus 10 can operate with low power consumption. For example, when corrosion has not progressed, the intervals between the drive periods during which the measurement unit 20 is driven are long, so the sensor apparatus 10 can operate for a long period of time without recharging. In the sensor system 1, the terminal apparatus 30 notifies a user of an alert, in response to the degree of corrosion. Therefore, the sensor apparatus 10 itself can detect the corrosive environment at an early stage and notify the user as necessary, without relying on the measurement timing of the operator.

In addition, the sensor apparatus 10 increases the frequency of measurement by the temperature and humidity sensor 12 in response to the progress of corrosion. Therefore, the sensor system 1 can identify a timing and time when corrosion becomes severe, a water intrusion route, and the like at an early stage. The sensor system 1 can simply detect critical corrosive environment by considering leaked radio waves from the second transmitter 14, which is covered by the shield 15 containing iron foil, as an alarm signal.

The present disclosure is not limited to the embodiment described above. For example, multiple blocks illustrated in the block diagram may be integrated, or one block may be divided. Multiple steps illustrated in the flowchart may be executed in parallel or in a different order, depending on the processing capacity of the apparatus executing each step or as necessary, instead of being executed sequentially according to the description. Other modifications are also possible within the scope of not deviating from the gist of the present disclosure.

## Claims

1. A sensor apparatus (10) comprising:
a sensor (12) configured to acquire a measurement value of a surrounding physical quantity;
an activation transmitter (13) configured to transmit an activation signal that is a signal indicating activation of the sensor apparatus (10); and
a timer (16) configured to determine transmission timings of transmitting the activation signal from the activation transmitter (13),
wherein
the activation transmitter (13) is configured to transmit, as the activation signal, a signal containing the measurement value acquired by the sensor at the transmission timings determined by the timer (16), and
the timer (16) is configured to reduce an interval between the transmission timings in response to variation in a resistance value of a resistor (167) whose resistance value varies in response to progress of corrosion.

2. The sensor apparatus (10) according to claim 1, wherein
the sensor (12) is activated immediately before start of the transmission and acquires the measurement value, and
the activation transmitter (13) is configured to transmit, as the activation signal, a signal containing the measurement value acquired by the sensor (12) immediately before transmission of the signal.

3. The sensor apparatus (10) according to any one of claims 1 to 2, wherein the activation transmitter (13) is configured to transmit the activation signal by wireless communication.

4. The sensor apparatus (10) according to any one of claims 1 to 3, further comprising:
a corrosion transmitter (14) configured to transmit, at the transmission timings by wireless communication, a corrosion signal that is a signal indicating that the corrosion has progressed to a certain degree; and
a shield (15) configured to block the corrosion signal transmitted from the corrosion transmitter (14),
wherein the shield (15) is configured to have a reduced function of blocking the corrosion signal in response to the progress of the corrosion.

5. The sensor apparatus (10) according to claim 4, wherein the shield (15) is made of iron.

6. The sensor apparatus (10) according to any one of claims 1 to 5, further comprising:
a rechargeable battery (7) configured to supply electric power to drive the timer (16); and
a power receiver (18) configured to receive electric power to recharge the battery (7).

7. A terminal apparatus (30) configured to be communicable with the sensor apparatus (10) according to any one of claims 1 to 6, the terminal apparatus (30) comprising a controller (11) configured to cause an output interface (35) to output an alert when a reception interval of the activation signal from the sensor apparatus (10) is less than a predetermined threshold value.

8. A terminal apparatus (30) configured to be communicable with the sensor apparatus (10) according to claim 6, the terminal apparatus (30) comprising a controller (11) configured to cause a power supply (36) to supply electric power to the sensor apparatus (10) when no signal has been received from the sensor apparatus (10) over a predetermined period of time.

9. A sensor system comprising:
the sensor apparatus (10) according to any one of claims 1 to 6; and
the terminal apparatus (30) according to claim 7.
